# EUROPÄISCHE PATENTSCHRIFT

(11) **EP 1 160 234 B1**
(45) Veröffentlichungstag und Bekanntmachung des Hinweises auf die Patenterteilung: **04.05.2005**
(21) Anmeldenummer: 01122277.5
(22) Anmeldetag: 30.12.1997
(51) Int. Cl.: C07C 45/50, C07C 47/02

(54) **Verfahren zur Herstellung von Aldehyden durch Hydroformylierung**
Process for the preparation of aldehydes by hydroformylation
Procédé pour la préparation d' aldéhydes par hydroformylation

(30) Priorität: 13.01.1997 DE 19700805
(43) Veröffentlichungstag der Anmeldung: 05.12.2001
(62) Teilanmeldung aus: 97954759.3
(73) Patentinhaber: Celanese Chemicals Europe GmbH, 60439 Frankfurt am Main (DE)
(72) Erfinder: Bogdanovic, Sandra, Dr., 14195 Berlin (DE); Bahrmann, Helmut, Dr., 46499 Hamminkeln (DE); Frohning, Carl Dieter, Dr., 46485 Wesel (DE); Wiebus, Ernst, 46147 Oberhausen (DE)

(56) Entgegenhaltungen:
- EP-A- 0 157 316
- FR-A- 2 314 910
- FR-A- 2 489 308
- US-A- 3 404 188

## Beschreibung

Die vorliegende Erfindung betrifft ein Verfahren zur Herstellung von Aldehyden durch Umsetzung von Buten oder Penten mit Wasserstoff und Kohlenmonoxid bei erhöhtem Druck in Anwesenheit einer Rhodium und sulfonierte Triarylphosphine als Katalysator enthaltenden wäßrigen Phase.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, hat in letzter Zeit Rhodium zunehmende Bedeutung erlangt. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von RhodiumKatalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls im Überschuß eingesetzte Liganden enthalten. Besonders bewährt haben sich als Liganden tertiäre Phosphine oder Phosphite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogenen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d. h. Olefine mit bis zu etwa 5 Kohlenstoffatomen im Molekül, beschritten werden.

Bei der Hydroformylierung langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischem Wege wird durch Anwendung in Wasser löslicher Katalysatorsysteme vermieden. Derartige Katalysatoren sind z. B. in der DE-PS 26 27 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d. h. ohne Destillation und damit ohne zusätzliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Der Einsatz wasserlöslicher Katalysatoren hat sich bei der Hydroformylierung niederer Olefine, insbesondere Propen und Buten, bewährt. Setzt man höhere Olefine wie Penten oder Hexen ein, geht jedoch die Umsetzungsgeschwindigkeit bereits merklich zurück. Die Wirtschaftlichkeit der Umsetzung in technischem Maßstab ist bei Einsatz von Olefinen mit mehr als vier Kohlenstoffatomen häufig nicht mehr im gewünschten Umfang gegeben.

Aus der EP-A1-0 805 139 ist ein Hydroformylierungsverfahren für höhere Olefine bekannt, das in einem polaren organischen Lösungsmittel, beispielsweise in einem Polyalkylenglykol, in Gegenwart von mono-sulfonierten tertiären organischen Phosphinen durchgeführt wird. Nach erfolgter Hydroformylierungsreaktion wird das Rohprodukt mit Wasser extrahiert, wobei das organische Reaktionsprodukt zurückbleibt und die Rhodiumverbindung, das mono-sulfonierte Phosphin sowie das polare organische Lösungsmittel in die wäßrige Phase übergehen. Nach der Wasserentfernung und dem Ansäuern bleibt das polare, organische Lösungsmittel, enthaltend die Rhodiumverbindung und das mono-sulfonierte Phosphin, zurück, das anschließend in den Reaktor zurückgefahren werden kann.

Um bei der Hydroformylierung höherer Olefine mittels wasserlöslicher Katalysatoren den Umsatz und/oder die Selektivität der Reaktion zu n-Aldehyden zu steigern, wird der Zusatz eines amphiphilen Reagenz (DE 31 35 127 A1) oder eines Lösungsvermittlers (DE 34 12 335 A1 ) empfohlen.

Sehr hohe Umsätze werden sowohl gemäß DE 31 35 127 A1 als auch gemäß DE 34 12 335 unter Verwendung quaternärer Ammoniumsalze, die einen langkettigen Alkylrest aufweisen, erhalten, während nichtionische Stoffe auf Basis Polyethylenglykol zu vergleichsweise niedrigen Umsätzen führen.

Wie aus der DE 31 35 127 Tabelle 7 hervorgeht, führt die Hydroformylierung von Dodecen-(1) mittels Rhodium und monosulfoniertem Triphenylphosphin (3-Ph₂PC₆H₄SO₃Na) ohne Zusatz eines amphiphilen Reagenz zu einem Umsatz von 56 % (Beispiel 77), während der Zusatz von C₁₂H₂₅(OCH₂CH₂)₂₃OH (="Brij 35") zu einer Minderung des Umsatzes auf 37 % (Beispiel 78) führt.

Gemäß DE 34 12 335 (Tabelle 4) führt die Hydroformylierung von Hexen mittels Rhodium und Trinatrium-tri(n-sulfophenyl)phosphin ohne Zusatz eines Lösungsvermittlers zu einem Umsatz von 36 % (Beispiel 10), während ein Zusatz von 2,5 % Triethylenglykol (Beispiel 14) bzw. von 5 % Polyglykol 200 (Beispiel 11) einen Umsatz von 43,5 % bzw. 43 % ergibt. Der Zusatz der Lösungsvermittler hat keine signifikante Steigerung des Umsatzes zu Folge und auch die Erhöhung der Menge von 2,5 % und 5% Lösungsvermittler bewirkt keine Erhöhung des Umsatzes. Ein sehr hoher Umsatz, nämlich 86 %, wird hingegen mit einem Zusatz von 2,5 % Trimethylhexadecylammoniumbromid erzielt.

Der Einsatz quaternärer Ammoniumsalze als amphiphiles Reagenz oder Lösungsvermittler ist allerdings wegen der schlechten biologischen Abbaubarkeit dieser Verbindungen nicht unproblematisch. So führt die Anwesenheit quaternärer Ammoniumsalze im Abwasser zu Schwierigkeiten bei Abwasseraufarbeitung.

Amphiphile Reagenzien und Lösungsvermittler dienen dazu, den Stofftransport zwischen den einzelnen Phasen und damit die Vermischbarkeit von wäßriger Katalysatorphase und organischer Phase zu begünstigen. Eine Steigerung der Vermischbarkeit von wäßriger Katalysatorphase und organischer Phase geht mit einer erhöhten Löslichkeit von organischer Phase in wäßriger Phase und von wäßriger Phase in organischer Phase einher. Auf diese Weise kann in zunehmendem Maße sowohl amphiphiles Reagenz und Lösungsvermittler als auch Rhodium und wasserlösliches Phosphin in die organische Phase gelangen und nach Phasentrennung mit der organischen Phase ausgetragen werden.

Ferner ist zu erwarten, daß mit steigender Vermischbarkeit von wäßriger Katalysatorphase und organischer Phase die für die Phasentrennung erforderliche Entmischung infolge der Bildung von Emulsionen oder Lösungen nicht mehr oder nicht in genügenden Umfange stattfindet. Eine entsprechende Steigerung der Vermischbarkeit ist insbesondere bei einer Erhöhung des Zusatzes amphiphiler Reagenzien und Lösungsvermittler zu erwarten.

Ein erhöhter Austrag von Rhodium, wasserlöslichem Phosphin und amphiphilem Reagenz oder Lösungsvermittler über die organische Phase ist ebenso wie eine herabgesetzte Entmischbarkeit der Phasen unerwünscht, da Rhodium, wasserlösliches Phosphin und amphiphiles Reagenz oder Lösungsvermittler in der wäßrigen Katalysatorphase verbleiben sollen und eine gute Entmischbarkeit eine unabdingbare Voraussetzung für die erforderliche, die Hydroformylierung abschließende Trennung von organischer und wäßriger Phase ist.

In Hinblick auf die voranstehenden Darlegungen besteht ein Bedarf, ein Verfahren bereitzustellen, das die vorstehend genannten Nachteile vermeidet und sich zudem auf einfache Weise technisch realisieren läßt.

Diese Aufgabe wird gelöst durch ein Verfahren zur Herstellung von Aldehyden. Es ist dadurch gekennzeichnet, daß man Buten in Anwesenheit einer Rhodium und sulfonierte Triarylphosphine als Katalysator und von 8 bis 20 Gew.-% einer Verbindung der Formel (1) R(OCH₂CH₂)ₙOR¹ enthaltenden wäßrigen Phase, wobei die Triarylphosphine wenigstens drei -(SO₃)M-Reste enthalten, bei denen M gleich oder verschieden ist und M für H, ein Alkalimetallion, ein Ammoniumion, ein quaternäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

Diese Aufgabe wird weiterhin gelöst durch ein Verfahren zur Herstellung von Aldehyden, dadurch gekennzeichnet, daß man Penten in Anwesenheit einer Rhodium und sulfonierte Triarylphosphine als Katalysator und von 8 bis 30 Gew.-% einer Verbindung der Formel (1) R(OCH₂CH₂)ₙOR¹ enthaltenden wäßrigen Phase, wobei die Triarylphosphine wenigstens drei -(SO₃)M-Reste enthalten, bei denen M gleich oder verschieden ist und M für H, ein Alkalimetallion, ein Ammoniumion, ein quaternäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

In Hinblick auf die vorstehend geschilderten Befunde der DE 34 12 335 (Tabelle 4, Beispiele 10, 14 und 11) und DE 31 35 127 (Tabelle 7, Beispiele 77 und 78) bei der Hydroformylierung von Hexen und Dodecen war es nicht zu erwarten, daß bei der Umsetzung von Buten oder Penten ein Zusatz von Verbindungen der Formel (1) R(OCH₂CH₂)ₙOR¹ in den voranstehend genannten Mengen zu einem deutlichen Anstieg des Umsatzes und gleichzeitig hoher Selektivität bezüglich der Bildung von n-Aldehyden führen würde.

Es ist ferner generell überraschend, daß bei der Hydroformylierung von Buten oder Penten sogar ein Zusatz vergleichsweise großer Mengen von Verbindungen der Formel (1) R(OCH₂CH₂)ₙOR¹ nicht einen signifikanten Anstieg von Rhodium und sulfoniertem Triarylphosphin in der organischen Phase und somit einen erhöhten Austrag des Katalysators über die organische Phase bewirkt.

Darüber hinaus war nicht zu erwarten, daß trotz der vergleichsweise großen Mengen von Verbindungen der Formel (1), die Entmischbarkeit von organischer Phase und wäßriger Katalysatorphase so hoch ist, daß eine schnelle Trennung von organischer Phase und wäßriger Katalysatorphase gewährleistet ist. Überraschenderweise werden schwertrennbare Emulsionen oder homogene, nicht trennbare Phasen respektive Lösungen nicht gebildet.

Die den Katalysator und die Verbindung der Formel (1) R(OCH₂CH₂)OR¹ enthaltende wäßrige Phase läßt sich auf vergleichsweise einfache Art herstellen, indem man ein wasserlösliches Rhodiumsalz, die sulfonierten Triarylphosphine und die Verbindung der Formel (1) in Wasser löst.

Geeignete Rhodiumsalze sind, ohne Anspruch auf Vollständigkeit zu erheben: Rhodium(III)sulfat, Rhodium(III)nitrat, Rhodium(III)carboxylate wie Rhodiumacetat, Rhodiumpropionat, Rhodiumbutyrat, Rhodium-2-ethylhexanoat.

Man kann die wäßrige Phase unmittelbar in die Hydroformylierung einsetzen oder sie zuvor einer Präformierung des Katalysators unter Reaktionsbedingungen unterwerfen und sie anschließend in präformierter Form zu verwenden.

Als olefinische Verbindung kann man Buten oder Penten einsetzen, insbesondere 1-Buten, technisch verfügbare Gemische, die im wesentlichen 1-Buten und 2-Buten enthalten, und 1-Penten.

Unter sulfonierten Triarylphosphinen werden Phosphine mit einem oder zwei Phosphoratomen verstanden, die je Phosphoratom drei Arylreste besitzen, die Arylreste gleich oder verschieden sind und für einen Phenyl-, Naphthyl-, Biphenyl-, Phenylnaphthyl- oder Binaphthyl-Rest, insbesondere Phenyl-, Biphenyl oder Binaphthyl-Rest stehen, die Arylreste mit dem Phosphoratom direkt oder über eine -(CH₂)ₓ-Gruppe, worin x für eine ganze Zahl von 1 bis 4, insbesondere 1 bis 2, bevorzugt 1, steht, verbunden sind, und wenigstens drei -(SO₃)M Reste enthalten, worin M gleich oder verschieden ist und für H, ein Alkalimetallion, ein Ammoniumion, ein quaternäres Ammoniumion, ein ½ Erdalkalimetallion oder ½ Zinkion, insbesondere ein Alkalimetallion, ein Ammoniumion oder quaternäres Ammoniumion, bevorzugt ein Alkalimetallion steht. Die -SO₃M-Reste sitzen üblicherweise als Substituenten an den Arylresten und verleihen den Triarylphosphinen die erforderliche Wasserlöslichkeit.

Man verwendet als sulfonierte Triarylphosphine mit einem Phosphoratom, Verbindungen der Formel (2) 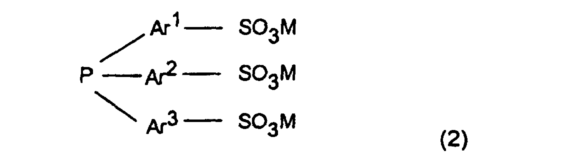 worin Ar¹, Ar² und Ar³ gleich oder verschieden sind und jeweils einen Phenyl- oder Naphthylrest, insbesondere einen Phenylrest, bedeuten, und M gleich oder verschieden, insbesondere gleich ist und für ein Alkalimetallion, ein Ammoniumion, ein quaternäres Ammoniumion oder ein ½ Erdkalkalimetallion oder ½ Zinkion, insbesondere ein Alkalimetallion oder Ammoniumion, bevorzugt ein Alkalimetallion, besonders bevorzugt ein Natriumion, steht.

Besonders geeignet ist Trinatrium-tri-(m-sulfophenyl)phosphin als sulfoniertes Triarylphosphin. Dieses Trinatriumsalz des Tri-(meta-sulfophenyl)phosphins enthält aufgrund seiner Herstellung durch Sulfonierung von Triphenylphosphin auch noch Anteile an mono- und disulfonierten Verbindungen.

Das Trinatrium-tri(m-sulfophenyl)phosphin entspricht der nachfolgenden Formel:

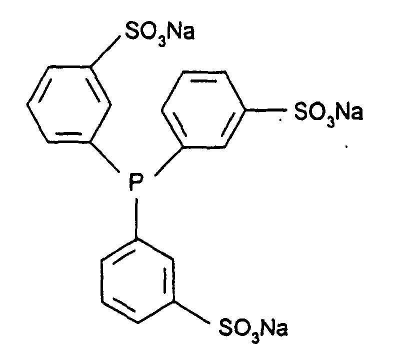

Die sulfonierten Triarylphosphine mit zwei Phosphoratomen können beispielsweise einen Rest -(CH₂)ₓ-Ar-Ar-(CH₂)ₓ- enthalten, worin x für eine ganze Zahl von 1 bis 4, insbesondere 1 bis 2, vorzugweise 1 steht, Ar-Ar Biphenyl oder Binaphthyl bedeutet, die -(CH₂)ₓ-Gruppe mit der einen Bindung jeweils in ortho-Stellung zu der die beiden Arylreste verbindenden Aryl-Arylbindung Ar-Ar steht und mit der anderen Bindung jeweils mit einem Phosphoratom, das jeweils zwei weitere, gleiche oder verschiedene Arylreste, insbesondere Phenylreste besitzt, verbunden ist. Diese zwei Phosphoratome enthaltenden Triarylphosphine weisen wenigstens drei -SO₃M-Reste, insbesondere vier bis acht -SO₃M-Reste, auf, worin M die vorstehend genannte Bedeutung besitzt. Die -SO₃M-Reste sitzen üblicherweise an den Arylresten des Restes -(CH₂)ₓ-Ar-Ar-(CH₂)ₓ- und an den zwei weiteren Arylresten, die mit dem Phosphor verbunden sind.

Beispiele für derartige, zwei Phosphoratome enthaltende, sulfonierte Triarylphosphine sind, ohne Anspruch auf Vollständigkeit zu erheben, durch die nachfolgenden Formeln (3) und (4) gegeben:

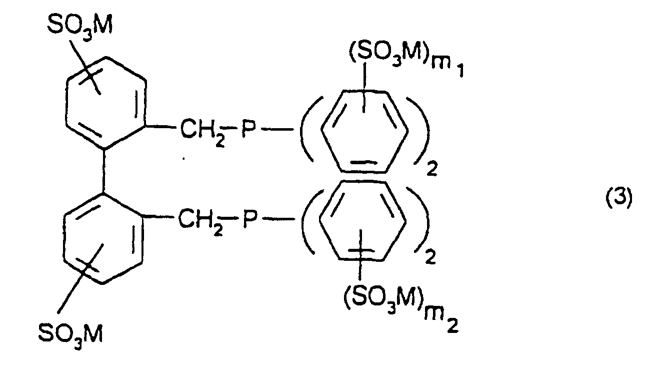

In (3) steht ein jedes m₁ und m₂ unabhängig voneinander für 0 oder 1, wobei die Verbindung der Formel (3) drei bis sechs -SO₃M Gruppen enthält.

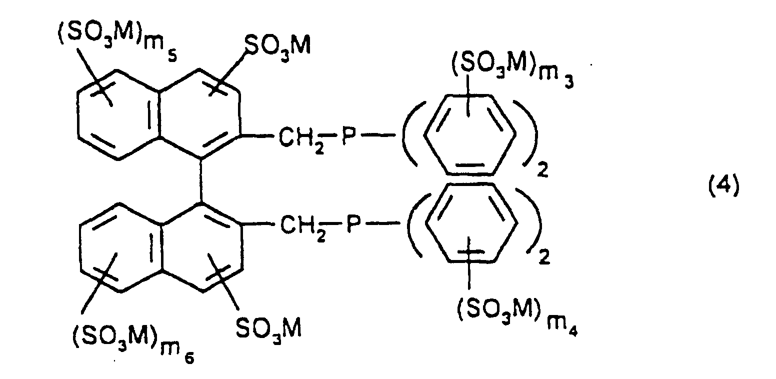

In (4) steht ein jedes m₃, m₄, m₅ und m₆ unabhängig voneinander für 0 oder 1, wobei die Verbindung der Formel (4) vier bis acht, insbesondere fünf bis sechs, -SO₃M-Gruppen enthält.

Aufgrund der Herstellung durch Sulfonierung der entsprechenden Phosphine der Formel (3a) und (4a), die keine -SO₃M-Gruppen enthalten, 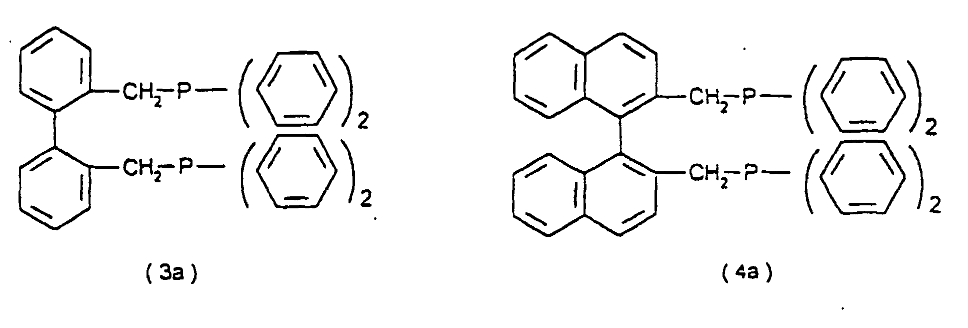 erhält man üblicherweise Gemische von Verbindungen (3) und (4) mit unterschiedlicher Anzahl von -SO₃M-Gruppen. So enthält eine Verbindung der Formel (3) oder (4), die beispielsweise drei SO₃M-Gruppen enthält, auch Verbindungen mit lediglich zwei -SO₃M-Gruppen aber auch Verbindungen mit vier oder fünf -SO₃M-Gruppen. Eine Verbindung der Formel (3) oder (4) mit beispielsweise fünf -SO₃M-Gruppen enthält üblicherweise auch Verbindungen mit lediglich drei oder vier -SO₃M-Gruppen, aber auch Verbindungen mit sechs oder sieben -SO₃M-Gruppen.

Verbindungen der Formel (3) besitzen maximal sechs -SO₃M-Gruppen, während Verbindungen der Formel (4) maximal acht -SO₃M-Gruppen aufweisen.

Aus diesem Grund gelangen in der Regel Mischungen von Verbindungen der Verbindungen (3) und (4) mit unterschiedlicher Anzahl von -SO₃M-Gruppen zum Einsatz.

Die vorstehend beschriebenen sulfonierten Triarylphosphine besitzen aufgrund ihrer Sulfonat-Reste eine für die Durchführung des Verfahrens ausreichende Löslichkeit in Wasser.

Man setzt die Rhodium und die Verbindungen der Formel (2) als Katalysator und die Verbindung der Formel (1) enthaltende wäßrige Phase üblicherweise entsprechend einer Menge von 2x10⁻⁶ bis 5x10⁻², insbesondere 5x10⁻⁵ bis 5x10⁻², bevorzugt 1x10⁻⁴ bis 1x10⁻³ Mol Rhodium pro Mol olefinischer Verbindung ein.

Die Rhodiummenge hängt auch von der Art der zu hydroformylierenden olefinischen Verbindung ab. Obgleich niedrigere Katalysatorkonzentrationen möglich sind, können sie sich im Einzelfall als nicht besonders zweckmäßig erweisen, da die Reaktionsgeschwindigkeit zu gering und daher nicht wirtschaftlich genug sein kann. Die obere Katalysatorkonzentration kann bis 1x10⁻¹ Mol Rhodium pro Mol olefinische Verbindung betragen. Durch vergleichsweise hohe Rhodiumkonzentrationen ergeben sich allerdings keine besonderen Vorteile.

Man setzt die Rhodium und sulfonierte Triarylphosphine als Katalysator und die Verbindung der Formel (1) R(OCH₂CH₂)ₙOR¹ enthaltende wäßrige Phase und die olefinische Verbindung üblicherweise im Volumenverhältnis 10:1 bis 1:10, insbesondere 5:1 bis 1:5, bevorzugt 2:1 bis 1:2 ein.

Man setzt Rhodium und sulfonierte Triarylphosphine im Molverhältnis 1:5 bis 1:2000 ein.

Verwendet man ein sulfoniertes Triarylphosphin mit einem Phosphoratom, beispielsweise eine Verbindung der Formel (2), so setzt man üblicherweise Rhodium und sulfoniertes Triarylphosphin im Molverhältnis 1:10 bis 1:1000, insbesondere 1:50 bis 1:200, bevorzugt 1:80 bis 1:120, ein.

Verwendet man ein sulfoniertes Triarylphosphin mit zwei Phosphoratomen (beispielsweise Verbindungen der Formel (3) und (4)), so setzt man Rhodium und sulfoniertes Triarylphosphin üblicherweise im Molverhältnis 1:5 bis 1:100, insbesondere 1:5 bis 1:50, bevorzugt 1:8 bis 1:15, ein.

Die wäßrige Phase enthält 20 bis 2000 ppm Rhodium. Falls man ein sulfoniertes Triarylphosphin mit einem Phosphoratom, beispielsweise Verbindungen der Formel (2), einsetzt, so verwendet man in den meisten Fällen eine wäßrige Phase, die 100 bis 1000, insbesondere 200 bis 500, bevorzugt 300 bis 400 ppm Rhodium enthält. Falls man ein sulfoniertes Triarylphosphin mit zwei Phosphoratomen, beispielsweise Verbindungen der Formel (3) und/oder (4) einsetzt, so verwendet man in den meisten Fällen eine wäßrige Phase, die 20 bis 500, insbesondere 30 bis 150, bevorzugt 40 bis 100 ppm Rhodium enthält.

Die Art der umzusetzenden olefinischen Verbindung kann in gewissem Umfange auch die Menge der einzusetzenden Verbindung der Formel (1) R(OCH₂CH₂)ₙOR¹ beeinflussen.

Setzt man als olefinische Verbindung Buten ein, so hat es sich häufig als sinnvoll erwiesen, die Umsetzung in Anwesenheit einer 8 bis 20 Gew.-% der Verbindung der Formel (1) enthaltenden wäßrigen Phase durchzuführen.

Setzt man als olefinische Verbindung Penten ein, so hat es sich häufig als sinnvoll erwiesen, die Umsetzung in Anwesenheit einer 8 bis 30 Gew.-% der Verbindung der Formel (1) enthaltenden wäßrigen Phase durchzuführen.

An dieser Stelle sei der Vollständigkeit halber erwähnt, daß es sich bei den Verbindungen der Formel (1) R(OCH₂CH₂)ₙOR¹, worin R für Wasserstoff, einen geradkettigen oder verzweigten Alkylrest mit 1 bis 4 Kohlenstoffatomen oder einen Hydroxyalkylrest mit 1 bis 4 Kohlenstoffatomen, insbesondere für Wasserstoff, einen Alkylrest mit 1 bis 2 Kohlenstoffatomen oder einen Hydroxyalkylrest mit 1 bis 3 Kohlenstoffatomen, bevorzugt für Wasserstoff, Methyl, Hydroxymethyl oder Hydroxypropyl und R¹ für Wasserstoff oder einen Methylrest, insbesondere Wasserstoff, steht, um Stoffe handelt, die sich in Wasser in ausreichendem Umfange lösen.

Es sei an dieser Stelle auf die nachfolgenden Verbindungen der Formel (1), worin R¹ für Wasserstoff steht, hingewiesen, die von besonderem Interesse sind.

Ohne Anspruch auf Vollständigkeit zu erheben, seien als Verbindungen der Formel R(OCH₂CH₂)ₙOH, Polyethylenglykol der Formel H(OCH₂CH₂)ₙOH mit einem mittleren Molgewicht von ungefähr 200 (PEG 200), 400 (PEG 400), 600 (PEG 600) oder 1000 (PEG 1000), Verbindungen der Formel CH₃(OCH₂CH₂)ₙOH mit einem mittleren Molgewicht von ungefähr 350 (M 350), 500 (M 500) oder 750 (M 750) oder Verbindungen der Formel CH₃CHOHCH₂(OCH₂CH₂)ₙOH mit einem mittleren Molgewicht von ungefähr 300 (300 PR), 450 (450 PR), 600 (600 PR) oder 1000 (1000PR), insbesondere Polyethylenglykol mit einem mittleren Molgewicht von ungefähr 400 (PEG 400) und 600 (PEG 600), eine Verbindung der Formel CH₃(OCH₂CH₂)ₙOH mit einem mittleren Molgewicht von 500 (M 500) oder eine Verbindung der Formel CH₃CHOHCH₂(OCH₂CH₂)ₙOH mit einem mittleren Molgewicht von 450 (450 PR) und 600 (600 PR) genannt.

Es ist unter PEG 200 ein Gemisch von Polyethylenglykolen der Formel H(OCH₂CH₂)ₙOH, worin n für eine ganze Zahl von 3 bis 6 steht, unter PEG 400 ein Gemisch von Polyethylenglykolen der Formel H(OCH₂CH₂)ₙOH, worin n für eine ganze Zahl von 7 bis 10 steht, unter PEG 600 ein Gemisch von Polyethylenglykolen der Formel H(OCH₂CH₂)ₙOH, worin n für eine ganze Zahl von 11 bis 16 steht und unter PEG 1000 ein Gemisch von Polyethylenglykolen der Formel H(OCH₂CH₂)ₙOH, worin n für eine ganze Zahl von 15 bis 30 steht, zu verstehen. Diesen Gemischen ist jeweils ein entsprechendes mittleres Molgewicht von etwa 200 (PEG 200), etwa 400 (PEG 400), etwa 600 (PEG 600) respektive etwa 1000 zuzuordnen.

Es ist unter M 350 ein Gemisch von Verbindungen der Formel CH₃(OCH₂CH₂)ₙOH, worin n für eine ganze Zahl von 5 bis 9 steht, unter M 500 ein Gemisch von Verbindungen der Formel CH₃(OCH₂CH₂)ₙOH, worin n für eine ganze Zahl von 9 bis 13 steht, unter M 750 ein Gemisch von Verbindungen der Formel CH₃(OCH₂CH₂)ₙOH, worin n für eine ganze Zahl von 12 bis 20 steht, zu verstehen. Diesen Gemischen ist jeweils ein entsprechendes mittleres Molgewicht von etwa 350 (M 350), etwa 500 (M 500) respektive 750 (M 750) zuzuordnen.

Es ist unter 300 PR ein Gemisch von Verbindungen der Formel R(OCH₂CH₂)ₙOH, worin R für einen β-Hydroxypropylrest CH₃CHOHCH₂- und n für eine ganze Zahl von 6 bis 9 steht, unter 450 PR ein Gemisch von Verbindungen der Formel R(OCH₂CH₂)ₙOH, worin R für einen β-Hydroxypropylrest CH₃CHOHCH₂- und n für eine ganze Zahl von 8 bis 14 steht, unter 600 PR ein Gemisch von Verbindungen der Formel R(OCH₂CH₂)ₙOH, worin R für einen β-Hydroxypropylrest CH₃CHOHCH₂- und n für eine ganze Zahl von 12 bis 20 steht, unter 1000 PR ein Gemisch von Verbindungen der Formel R(OCH₂CH₂)ₙOH, worin R für einen β-Hydroxypropylrest CH₃CHOHCH₂- und n für eine ganze Zahl von 18 bis 26 steht, zu verstehen. Diesen Gemischen ist jeweils ein entsprechendes mittleres Molgewicht von etwa 300 (300 PR), etwa 450 (450 PR), etwa 600 (600 PR) respektive etwa 1000 (1000 PR) zuzuordnen.

In einer Reihe von Fällen hat es sich bewährt, ein Polyethylenglykol der Formel H(OCH₂CH₂)ₙOH, worin n für eine ganze Zahl von 3 bis 50, insbesondere 4 bis 30, bevorzugt 5 bis 20, besonders bevorzugt 6 bis 12, steht, als Verbindung der Formel (1) einzusetzen.

Es hat sich auch bewährt, eine Verbindung (Halbether) der Formel R(OCH₂CH₂)ₙOH, worin R für einen Methylrest oder β-Hydroxypropylrest und n für eine ganze Zahl von 3 bis 50, insbesondere 4 bis 30, bevorzugt 5 bis 20 steht, als Verbindung der Formel (1) einzusetzen.

Es lassen sich auch beliebige Mischungen der Verbindungen der Formel (1), nämlich Polyethylenglykole, Polyethylenglykolether (Halbether) und Polyethylenglykoldiether einsetzen.

Man führt die Umsetzung in Gegenwart von Wasserstoff und Kohlenmonoxid durch. Das Molverhältnis von Wasserstoff zu Kohlenmonoxid kann in weiten Grenzen gewählt werden und liegt üblicherweise bei 1:10 bis 10:1, insbesondere 5:1 bis 1:5, bevorzugt 2:1 bis 1:2, besonders bevorzugt 1,2:1 bis 1:1,2. Besonders einfach gestaltet sich das Verfahren, wenn man Wasserstoff und Kohlenmonoxid im Molverhältnis 1:1 oder annähernd 1:1 einsetzt.

Für viele Fälle reicht es aus, die Umsetzung bei einer Temperatur von 50 bis 150, inbesondere 100 bis 140°C durchzuführen.

In einer Vielzahl von Fällen hat es sich als nützlich erwiesen, die Umsetzung bei einem Druck von 10 bis 200, insbesondere 20 bis 150, bevorzugt 30 bis 80 bar durchzuführen.

Während der Umsetzung ist sicherzustellen, daß für eine gute Durchmischung von organischer Phase, wäßriger Phase und Kohlenmonoxid/Wasserstoff gesorgt wird. Dies kann beispielsweise durch intensives Rühren und/oder Umpumpen von organischer und wäßriger Phase bewirkt werden. In der organischen Phase befinden sich üblicherweise die olefinische Verbindung, die erzeugten Aldehyde sowie geringe Anteile der wäßrigen Phase, während die wäßrige Phase üblicherweise Rhodium, die sulfonierten Triarylphosphine, die Verbindung der Formel (1), Wasser und geringe Anteile der organischen Phase enthält.

Es sei an dieser Stelle nochmals darauf hingewiesen, daß die Reaktionsbedingungen, insbesondere Rhodiumkonzentration, Druck und Temperatur auch von der Art der zu hydroformylierenden olefinischen Verbindung abhängen. Vergleichsweise reaktive olefinische Verbindungen erfordern geringe Rhodiumkonzentrationen, niedrige Drücke und niedrige Temperaturen. Hingegen benötigt die Umsetzung relativ reaktionsträger olefinischer Verbindungen größere Rhodiumkonzentrationen, höhere Drücke und höhere Temperaturen.

Das Verfahren läßt sich mit besonders gutem Erfolg ausführen, wenn man eine α-olefinische Verbindung einsetzt. Es lassen sich jedoch auch andere olefinische Verbindungen mit innenständigen Kohlenstoff-Kohlenstoff-Doppelbindungen mit guten Ergebnissen umsetzen.

Das Hydroformylierungsgemisch wird nach Abschluß der Umsetzung durch Druckentspannung von Kohlenmonoxid und Wasserstoff befreit und das Reaktionsprodukt, gegebenenfalls nach Abkühlen, von der den Katalysator und die Verbindung der Formel (1) enthaltenden wäßrigen Phase durch Phasentrennung abgetrennt.

Die den Katalysator und die Verbindung der Formel (1) enthaltende wäßrige Phase kann wieder in das erfindungsgemäße Verfahren zurückgeführt werden, während die abgetrennte, das Reaktionsprodukt enthaltende organische Phase, beispielsweise durch fraktionierte Destillation, aufgearbeitet wird.

Das Verfahren läßt sich kontinuierlich und diskontinuierlich durchführen.

Die nachfolgenden Beispiele beschreiben die Erfindung näher, ohne sie zu beschränken.

### Experimenteller Teil:

### 1. Hydroformylierung von 1-Penten

### Beispiel 1a) (Vergleichsbeispiel zu Beispiel 1b) bis 1d) ohne Zusatz von Polyethylenglykol)

### I Herstellung der Katalysatorphase und Präformierung

60 mg (0,233 mmol) Rhodium(III)acetat werden in 39 ml einer 0,6 M wäßrigen Lösung von Trinatrium-tri-(m-sulfophenyl)phosphin (Na-TPPTS) entsprechend einem Molverhältnis Rhodium zu Ligand von 1:100 und 21 ml entgastem destillierten Wasser gelöst und in einen 200 ml Stahlautoklav unter Stickstoffstrom gegeben. Diese Katalysatorlösung wird unter Rühren bei 25 bar Synthesegasdruck (CO/H₂ = 1/1) während 3 Stunden auf 125 °C erwärmt, wobei die Lösung eine gelbe Farbe annimmt.

### II Hydroformylierung

Zu der präformierten Katalysatorlösung aus I werden bei einem Reaktionsdruck von 30 bar und bei 125 °C 26,3 ml (240 mmol) 1-Penten mittels eines vorgeschalteten 200 ml Stahlautoklaven mit leichtem Überdruck zugegeben. Das Verhältnis von Olefin zu Rhodium beträgt 1039 :1. Die Hydroformylierungsreaktion wird durch Einschalten des Magnetrührers in Gang gesetzt. Während einer Reaktionszeit von 3 Stunden wird die Temperatur auf 125 °C gehalten und der Reaktionsdruck in einem Druckband von +/- 2 bar durch manuelles Zudosieren von Synthesegas konstant gehalten. Nach Ablauf von 3 Stunden werden Rührer und Heizung abgeschaltet, der Autoklav wird auf 40 bis 50 °C abgekühlt und die obere Produktphase von der Katalysatorphase in einem Scheidetrichter getrennt. Produktphase und Katalysatorphase werden gewogen. Die Zusammensetzung der Produktphase wird mittels Gaschromatographie und ¹H-NMR-Spektroskopie ermittelt und aus der Zusammensetzung die Ausbeute an Hydroformylierungsprodukten und das Verhältnis von n-Hexanal zu iso-Hexanal (2-Methylpentanal) bestimmt. Der Rhodiumgehalt der organischen Phase wird elementaranalytisch mittels Graphitrohr-Atomabsorptionsspektrometrie nach Probenaufschluß bestimmt. Die Ausbeute an Hydroformylierungsprodukten beträgt 49,4 %, das n/iso-Verhältnis beträgt 96:4. Die organische Phase enthält 0,05 ppm Rh. (Beispiel 1a) in Tabelle 1)

### Beispiel 1b)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) gelöst. Zu dieser Lösung werden 5 ml entgastes Polyethylenglykol 400 gegeben und die Lösung auf 60 ml Gesamtvolumen aufgefüllt. Diese Katalysatorphase wird unter Stickstoffstrom in einen 200 ml Stahlautoklaven gegeben und unter Rühren bei 25 bar Synthesegasdruck (CO/H₂ = 1/1) während 3 Stunden auf 125°C erwärmt.

### II Hydroformylierung

Zu der präformierten Katalysatorlösung aus I werden analog Beispiel 1a) 30 ml (240 mmol) 1-Penten zugegeben. Die Hydroformylierung wird analog Beispiel 1a) bei 125°C und bei 30 bar Synthesegas durchgeführt. Die Produktphase wird analog Beispiel 1a) analysiert. Die Ausbeute an Hydroformylierungsprodukt beträgt 70,1 %, das n/iso-Verhältnis beträgt 96:4. (Beispiel 1b) in Tabelle 1).

### Beispiel 1c)

Es wird verfahren wie in Beispiel 1 b), jedoch mit dem Unterschied, daß der Katalysatorphase anstelle von 5 ml entgastem Polyethylenglykol 400 nunmehr 7 ml entgastes Polyethylenglykol 400 zugesetzt werden und das Gesamtvolumen der Katalysatorphase auf 60 ml aufgefüllt wird. Die Präformierungs- und Hydroformylierungsbedingungen sind identisch mit Beispiel 1b). Die Ausbeute an Hydroformylierungsprodukt beträgt 81,1 %, das n/iso-Verhältnis beträgt 96:4. Die organische Phase enthält 0,16 ppm Rh. (Beispiel 1c) in Tabelle 1).

### Beispiel 1d)

Es wird verfahren wie in Beispiel 1 b), jedoch mit dem Unterschied, daß der Katalysatorphase 10 ml entgastes Polyethylenglykol 400 zugesetzt werden und das Gesamtvolumen der Katalysatorphase auf 60 ml aufgefüllt wird. Die Präformierungsund Hydroformylierungsbedingungen sind identisch mit Beispiel 1 b) mit dem Unterschied, daß die Reaktionsdauer der Hydroformylierung 210 min (3,5 Stunden) beträgt. Die Ausbeute an Hydroformylierungsprodukt beträgt 88,0 %, das n/iso-Verhältnis beträgt 95:5. Die organische Phase enthält 0,08 ppm Rh. (Beispiel 1d) in Tabelle 1).

### Beispiel 1 e)

Es wird verfahren wie in Beispiel 1b), jedoch mit dem Unterschied, daß der Katalysatorphase 21 ml entgastes Polyethylenglykol 400 zugesetzt wird. Die Präformierungs- und Hydroformylierungsbedingungen sind identisch mit Beispiel 1b). Die Ausbeute an Hydroformylierungsprodukt beträgt 87,3 %, das n/iso-Verhältnis beträgt 91:9. Die organische Phase enthält 0,85 ppm Rh. (Beispiel 1e) in Tabelle 1).

### Beispiel 1f) ohne Zusatz einer Verbindung der Formel (1)

Es wird verfahren wie in Beispiel 1a), jedoch mit dem Unterschied, daß die Hydroformylierungsreaktion bei 50 bar Synthesegasdruck durchgeführt wird. Die Ausbeute an Hydroformylierungsprodukt beträgt 74,8 %, das n/iso-Verhältnis beträgt 96:4. Die organische Phase enthält 0,03 ppm Rh. (Beispiel 1f) in Tabelle 1).

### Beispiel 1g)

Es wird verfahren wie in Beispiel 1f), jedoch mit dem Unterschied, daß der Katalysatorphase 5 ml entgastes Polyethylenglykol 400 zugesetzt werden und das Gesamtvolumen der Katalysatorphase auf 60 ml aufgefüllt wird. Die Präformierungsund Hydroformylierungsbedingungen sind identisch mit Beispiel 1f). Die Ausbeute an Hydroformylierungsprodukt beträgt 84,9 %, das n/iso-Verhältnis beträgt 95:5. (Beispiel 1g) in Tabelle 1).

### Beispiel 1 h) (wie Beispiel 1 g) mit längerer Reaktionszeit)

Es wird verfahren wie in Beispiel 1g), jedoch mit dem Unterschied, daß die Hydroformylierungsreaktion 240 min (4 Stunden) durchgeführt wird. Die Ausbeute an Hydroformylierungsprodukt beträgt 88,4 %, das n/iso-Verhältnis beträgt 96:4. Die organische Phase enthält 0,09 ppm Rh (Beispiel 1h) in Tabelle 1).

### Beispiel 1i)

Es wird verfahren wie in Beispiel 1f), jedoch mit dem Unterschied, daß der Katalysatorphase 7 ml entgastes Polyethylenglykol 400 zugesetzt werden und das Gesamtvolumen der Katalysatorphase auf 60 ml aufgefüllt wird. Die Präformierungsund Hydroformylierungsbedingungen sind identisch mit Beispiel 1f). Die Ausbeute an Hydroformylierungsprodukt beträgt 84,8 %, das n/iso-Verhältnis beträgt 95:5. (Beispiel 1i) in Tabelle 1).

### Beispiel 1j)

Es wird verfahren wie in Beispiel 1f), jedoch mit dem Unterschied, daß der Katalysatorphase 10 ml entgastes Polyethylenglykol 400 zugesetzt werden und das Gesamtvolumen der Katalysatorphase auf 60 ml aufgefüllt wird. Die Präformierungsund Hydroformylierungsbedingungen sind identisch mit Beispiel 1f). Die Ausbeute an Hydroformylierungsprodukt beträgt 87,5 %, das n/iso-Verhältnis beträgt 94:6. Die organische Phase enthält 0,28 ppm Rh (Beispiel 1j) in Tabelle 1).

### Beispiel 1k) (Verwendung einer Verbindung der Formel CH₃(OCH₂CH₂)ₙOH).

Es wird verfahren wie in Beispiel 1g), jedoch mit dem Unterschied, daß anstelle von 5 ml Polyethylenglykol 400 das gleiche Volumen (5 ml) einer Verbindung der Formel CH₃(OCH₂CH₂)ₙOH, n = 5 bis 9 (Handelsprodukt der Fa. Hoechst, Bezeichnung M 350) der Katalysatorphase zugesetzt werden und das Gesamtvolumen der Katalysatorphase auf 60 ml aufgefüllt wird. Die Präformierungs- und Hydroformylierungsbedingungen sind identisch mit Beispiel 1g). Die Ausbeute an Hydroformylierungsprodukt beträgt 84,3 %, das n/iso-Verhältnis beträgt 95:5. Die organische Phase enthält 0,07 ppm Rh (Beispiel 1k) in Tabelle 1).

### Beispiel 1l) (Verwendung einer Verbindung der Formel CH₃CHOHCH₂(OCH₂CH₂)ₙOH (n = 8 bis 14)

Es wird verfahren wie in Beispiel 1g), jedoch mit dem Unterschied, daß anstelle von 5 ml Polyethylenglykol 400 (PEG 400) das gleiche Volumen (5 ml) einer Verbindung der Formel CH₃CHOHCH₂(OCH₂CH₂)ₙOH, n = 8 bis 14 (Handelsprodukt der Fa. Hoechst, Bezeichnung 450PR) der Katalysatorphase zugesetzt werden und das Gesamtvolumen der Katalysatorphase auf 60 ml aufgefüllt wird. Die Präformierungsund Hydroformylierungsbedingungen sind identisch mit Beispiel 1g). Die Ausbeute an Hydroformylierungsprodukt beträgt 84,0 %, das n/iso-Verhältnis beträgt 95:5. Die organische Phase enthält 0,09 ppm Rh (Beispiel 11) in Tabelle 1).

Beispiele 1 g), 1k) und 1l) zeigen, daß bei Verwendung unterschiedlicher Verbindungen der Formel (1) vergleichbare Ergebnisse hinsichtlich Ausbeute an Hydroformylierungsprodukten, Selektivität und Rhodiumgehalt der organischen Phase erhalten werden.

### Beispiel 1 m) (Verwendung einer Verbindung der Formel H(OCH₂CH₂)₃OH (Triethylenglykol))

Es wird verfahren wie in Beispiel 1g), jedoch mit dem Unterschied, daß anstelle von 5 ml Polyethylenglykol 400 (PEG 400) das gleiche Volumen Triethylenglykol eingesetzt werden und das Gesamtvolumen der Katalysatorphase auf 60 ml aufgefüllt wird. Die Präformierungs- und Hydroformylierungsbedingungen sind identisch mit Beispiel 1g). Die Ausbeute an Hydroformylierungsprodukt beträgt 79,8 %, das n/iso-Verhältnis beträgt 94:6. Die organische Phase enthält 0,06 ppm Rh (Beispiel 1m) in Tabelle 1).

### Beispiel 1n) (Verwendung einer Verbindung der Formel CH₃(OCH₂CH₂)ₙOCH₃ (n = 3 bis 6)

Es wird verfahren wie in Beispiel 1g), jedoch mit dem Unterschied, daß anstelle von 5 ml Polyethylenglykol 400 (PEG 400) das gleiche Volumen einer Verbindung der Formel CH₃(OCH₂CH₂)ₙOCH₃ (n = 3 bis 6, Polyethylenglykoldimethylether) eingesetzt werden und das Gesamtvolumen der Katalysatorphase auf 60 ml aufgefüllt wird. Die Präformierungs- und Hydroformylierungsbedingungen sind identisch mit Beispiel 1g). Die Ausbeute an Hydroformylierungsprodukt beträgt 85,9 %, das n/iso-Verhältnis beträgt 95:5. (Beispiel 1n) in Tabelle 1).

Versuche mit Verbindungen der Formel (4) als Triarylphosphanliganden mit zwei Phosphoratomen.

### Beispiel 1 o) (Vergleichsbeispiel zu Beispiel 1p) und 1q) ohne Zusatz einer Verbindung der Formel (1))

### I Herstellung der Katalysatorphase und Präformierung

Die Katalysatorphase wird aus 7,5 mg (0,028 mmol) Rhodium(III)acetat, 1,8 ml einer 0,162 molaren Lösung von sulfoniertem 2,2'-Bis(diphenylphosphinomethyl)-1,1'binaphthalin (Na-BINAS) gemäß Formel (4) mit einer mittleren Anzahl von Sulfonatgruppen von 4 bis 7 entsprechend einem Molverhältnis Rhodium zu Ligand von 1:10 und 58 ml entgastem destillierten Wasser angesetzt und in einen 200 ml Stahlautoklav unter Stickstoffstrom gegeben. Diese Katalysatorlösung wird unter Rühren bei 25 bar Synthesegasdruck (CO/H₂ = 1/1) während 3 Stunden auf 125°C erwärmt.

### II Hydroformylierung

Die Hydroformylierungsreaktion wird bei einem Synthesegasdruck von 50 bar, die Aufarbeitung und Analytik der organischen Phase wird analog Versuch 1f) durchgeführt. Die Ausbeute an Hydroformylierungsprodukten beträgt 76,1 %, das n/iso-Verhältnis beträgt 98:2. (Beispiel 1o) in Tabelle 1).

### Beispiel 1 p)

Es wird verfahren wie in Beispiel 1o), jedoch mit dem Unterschied, daß der Katalysatorphase 5 ml entgastes Polyethylenglykol 400 zugesetzt werden und das Gesamtvolumen der Katalysatorphase auf 60 ml aufgefüllt wird. Die Ausbeute an Hydroformylierungsprodukt beträgt 76,1 %, das n/iso-Verhältnis beträgt 98:2 (Beispiel 1p) in Tabelle 1).

### Beispiel 1q)

Es wird verfahren wie in Beispiel 10), jedoch mit dem Unterschied, daß der Katalysatorphase 10 ml einer Verbindung der Formel CH₃(OCH₂CH₂)ₙOH, n = 9 bis13 (Handelsprodukt der Fa. Hoechst, Bezeichnung M 500) zugesetzt werden und das Gesamtvolumen der Katalysatorphase auf 60 ml aufgefüllt wird. Die Ausbeute an Hydroformylierungsprodukt beträgt 75,7 %, das n/iso-Verhältnis beträgt 98:2. (Beispiel 1q) in Tabelle 1).

### 2. Hydroformylierung von 1-Buten

### Beispiel 2a) (Vergleichsbeispiel zu Beispiel 2b) bis 2g) ohne Zusatz eines Additivs der Formel (1)

### I Herstellung der Katalysatorphase und Präformierung

60 mg (0,233 mmol) Rhodium(III)acetat werden in 39 ml einer 0,6 M wäßrigen Lösung von Trinatrium-tri-(m-sulfophenyl)phosphin (Na-TPPTS) entsprechend einem Molverhältnis Rhodium zu Ligand von 1 :100 und 21 ml entgastem destillierten Wasser gelöst und in einen 200 ml Stahlautoklav unter Stickstoffstrom gegeben. Diese Katalysatorlösung wird unter Rühren bei 25 bar Synthesegasdruck (CO/H₂ = 1/1) während 3 Stunden auf 125 °C erwärmt, wobei die Lösung eine gelbe Farbe annimmt.

### II Hydroformylierung

Zu der präformierten Katalysatorlösung aus I werden bei einem Reaktionsdruck von 30 bar und bei 125 °C 12,46 g (224 mmol) flüssiges 1-Buten mittels eines vorgeschalteten 200 ml Stahlautoklaven mit Überdruck zugegeben. (Die genaue Menge wird durch Differenzwägung ermittelt.) Das Verhältnis von Olefin zu Rhodium beträgt 950 :1. Die Hydroformylierungsreaktion wird durch Einschalten des Magnetrührers in Gang gesetzt. Während einer Reaktionszeit wird die Temperatur auf 125°C gehalten und der Reaktionsdruck in einem Druckband von +/- 2 bar durch manuelles Zudosieren von Synthesegas konstant gehalten. Die Reaktion wird nach 120 min beendet, da kein Synthesegas mehr aufgenommen wird. Der Rührer und die Heizung werden abgeschaltet, der Autoklav auf 40 bis 50°C abgekühlt und die obere Produktphase von der Katalysatorphase in einem Scheidetrichter getrennt. Die Ausbeute an Hydroformylierungsprodukten wird durch Auswaage und gaschromatographische Analyse der organischen Phase ermittelt, das Verhältnis von n-Pentanal zu iso-Pentanal (2-Methylbutanal) ebenfalls durch Gaschromatographie bestimmt.

Die Dauer der Hydroformylierungsreaktion ist in dieser Reihe von Beispielen ein Maß für die Hydroformylierungsgeschwindigkeit. Sie beträgt in diesem Beispiel 120 Minuten. Die Ausbeute an Hydroformylierungsprodukten beträgt 88,1 %, das n/iso-Verhältnis beträgt 96:4. Die organische Phase enthält 0,07 ppm Rh. (Beispiel 2a) in Tabelle 2)

### Beispiel 2b)

### I Herstellung der Katalysatorphase und Präformierung

60 mg (0,233 mmol) Rhodium(III)acetat werden in 39 ml einer 0,6 M wäßrigen Lösung von Trinatrium-tri(m-sulfophenyl)phosphin (Na-TPPTS) gelöst. Zu dieser Lösung werden 3 ml entgastes Polyethylenglykol 400 gegeben und die Lösung auf 60 ml Gesamtvolumen aufgefüllt. Diese Katalysatorphase wird unter Stickstoffstrom in einen 200 ml Stahlautoklaven gegeben und unter Rühren bei 25 bar Synthesegasdruck (CO/H₂ = 1/1) während 3 Stunden bei 125°C präformiert.

### II Hydroformylierung

Zu der präformierten Katalysatorlösung aus I werden 15,56 g (277 mmol) 1-Buten entsprechend einem Verhältnis von Olefin zu Rhodium von 1186:1 zugegeben. Die Hydroformylierung wird analog Beispiel 2a) bei 125°C und bei 30 bar Synthesegas durchgeführt. Nach 2 h erfolgt keine Druckaufnahme mehr. Die Ausbeute an Hydroformylierungsprodukt beträgt 87,2%, das n/iso-Verhältnis beträgt 96:4. Die organische Phase enthält 0,07 ppm Rh. In der gleichen Reaktionszeit werden alsobei gleichzeitig um 23,6 % erhöhter Menge an 1-Buten - 22 % mehr 1-Buten zu Hydroformylierungsprodukten umgesetzt, ohne daß die n/iso-Selektivität verändert wird und der Rhodiumgehalt der organischen Phase steigt. (Beispiel 2b) in Tabelle 2).

### Beispiel 2c)

Die Katalysatorphase wird analog Beispiel 2a) hergestellt und präformiert mit dem Unterschied, daß 6 ml entgastes Wasser durch 6 ml entgastes Polyethylenglykol ersetzt werden. Nach der Präformierung werden 13,34 g (238 mmol) 1-Buten entsprechend einem Verhältnis von Olefin zu Rhodium von 1017:1 zugesetzt. Die Reaktion ist bereits nach 90 min vollendet. Die Ausbeute an Hydroformylierungsprodukt beträgt 85,9 %, das n/iso-Verhältnis beträgt 96:4. Pro Zeiteinheit werden also unter Berücksichtigung der im Vergleich zu Beispiel 2a) erhöhten Menge 1-Buten und der geringeren Reaktionsdauer, 38 % mehr 1-Buten zu Hydroformylierungsprodukten umgesetzt als in Beispiel 2a). (Beispiel 2c) in Tabelle 1).

### Beispiel 2d)

Die Katalysatorphase wird analog Beispiel 2a) hergestellt und präformiert mit dem Unterschied, daß 9 ml entgastes Wasser durch 9 ml entgastes Polyethylenglykol ersetzt werden. Nach der Präformierung werden 13,89 g (247 mmol) 1-Buten entsprechend einem Verhältnis von Olefin zu Rhodium von 1058:1 zugesetzt. Die Hydroformylierungsreaktion ist bereits nach 60 min vollendet. Die Ausbeute an Hydroformylierungsprodukt beträgt 89,3 %, das n/iso-Verhältnis beträgt 94:6. Pro Zeiteinheit werden also unter Berücksichtigung der im Vergleich zu Beispiel 2a) höhten Menge 1-Buten und der geringeren Reaktionsdauer 2,2 mal soviel 1-Buten zu Hydroformylierungsprodukten umgesetzt als in Beispiel 2a). Der Rhodiumgehalt in der organische Phase beträgt 0,2 ppm (Beispiel 2d) in Tabelle 2).

### Beispiel 2e)

Die Katalysatorphase wird analog Beispiel 2a) hergestellt und präformiert, jedoch mit dem Unterschied, daß 12 ml entgastes Wasser durch 12 ml entgastes Polyethylenglykol 400 ersetzt werden. Nach der Präformierung werden 13,57 g (242 mmol) 1-Buten entsprechend einem Verhältnis von Olefin zu Rhodium von 1034:1 zugesetzt. Die Hydroformylierungsreaktion ist bereits nach 45 min vollendet. Die Ausbeute an Hydroformylierungsprodukt beträgt 88,3 %, das n/iso-Verhältnis beträgt 94:6. Pro Zeiteinheit werden also unter Berücksichtigung der im Vergleich zu Beispiel 2a) erhöhten Menge 1-Buten und der geringeren Reaktionsdauer 2,9 mal soviel 1-Buten zu Hydroformylierungsprodukten umgesetzt als in Beispiel 2a). (Beispiel 2e) in Tabelle 2).

### Beispiel 2f) (Verwendung einer Verbindung der Formel CH₃(OCH₂CH₂)ₙOH, n = 9 bis 13)

Die Katalysatorphase wird analog Beispiel 2d) hergestellt und präformiert mit dem Unterschied, daß anstelle von 9 ml entgastem Polyethylenglykol 400 das gleiche Volumen einer Verbindung der Formel CH₃(OCH₂CH₂)ₙOH, n = 9 bis 13 (Handelsprodukt der Fa. Hoechst, Bezeichnung M 500) eingesetzt wird. Nach der Präformierung werden 13,52 g (241 mmol) 1-Buten entsprechend einem Verhältnis von Olefin zu Rhodium von 1031:1 zugesetzt. Die Hydroformylierungsreaktion ist nach 60 min vollendet. Die Ausbeute an Hydroformylierungsprodukt beträgt 87,1 %, das n/iso-Verhältnis beträgt 95:5. Pro Zeiteinheit werden also also unter Berücksichtigung der im Vergleich zu Beispiel 2a) erhöhten Menge 1-Buten und der geringen Reaktionsdauer 2,13 mal soviel 1-Buten zu Hydroformylierungsprodukten umgesetzt als in Beispiel 2a). (Beispiel 2f) in Tabelle 2).

### Beispiel 2g) (Verwendung einer Verbindung der Formel CH₃(OCH₂CH₂)ₙOCH₃, n = 3 bis 6)

Die Katalysatorphase wird analog Beispiel 2d) hergestellt und präformiert mit dem Unterschied, daß anstelle von 9 ml entgastem Polyethylenglykol 400 das gleiche Volumen einer Verbindung der Formel CH₃(OCH₂CH₂)ₙOCH₃, n = 3 bis 6 eingesetzt wird. Nach der Präformierung werden 12,41g (221 mmol) 1-Buten entsprechend einem Verhältnis von Olefin zu Rhodium von 946:1 zugesetzt. Die Hydroformylierungsreaktion ist nach 50 min vollendet. Die Ausbeute an Hydroformylierungsprodukt beträgt 85,9 %, das n/iso-Verhältnis beträgt 95:5. Pro Zeiteinheit werden also also unter Berücksichtigung der geringen Reaktionsdauer 40,0 % mehr 1-Buten zu Hydroformylierungsprodukten umgesetzt als in Beispiel 2a). (Beispiel 2g) in Tabelle 2).

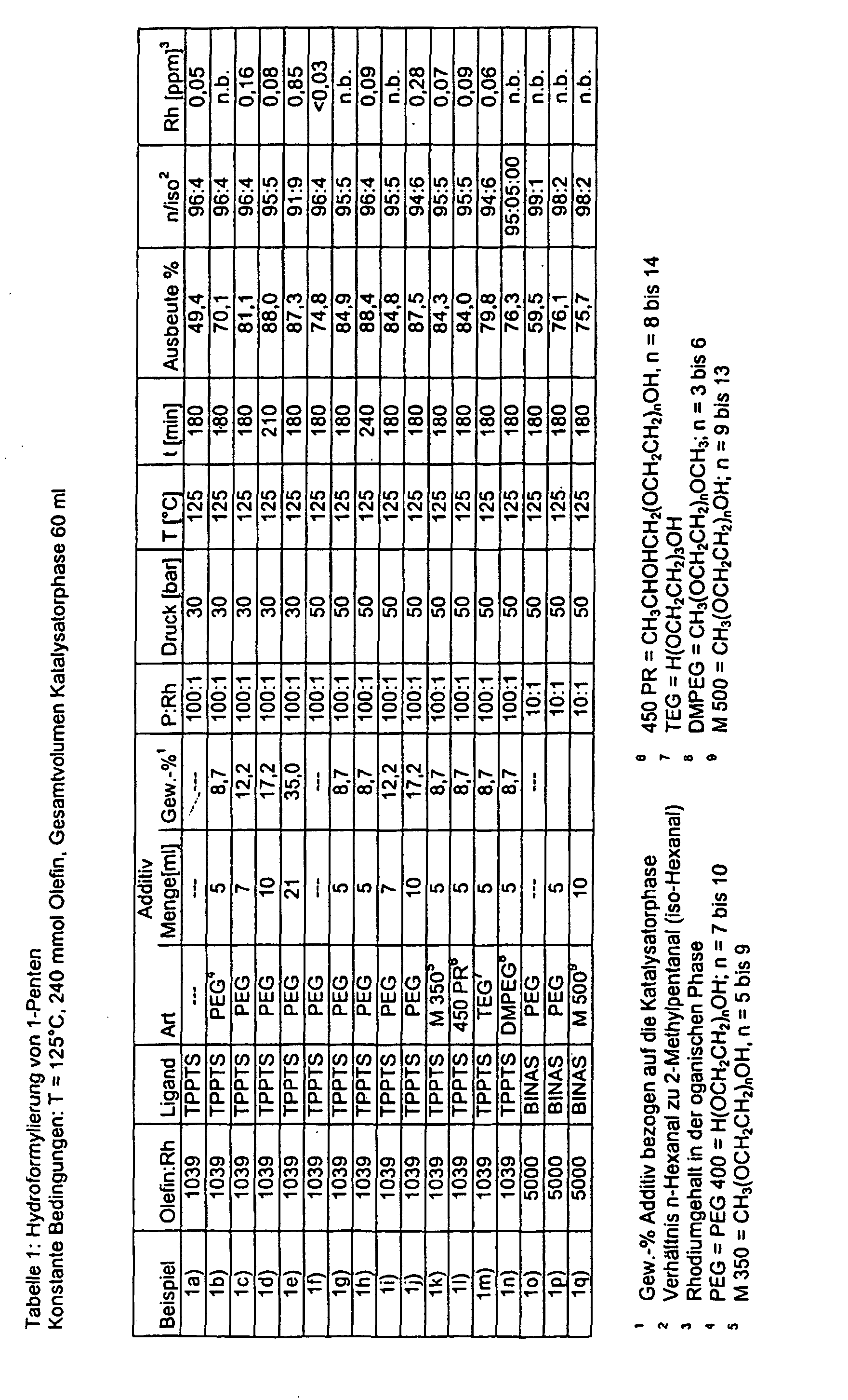

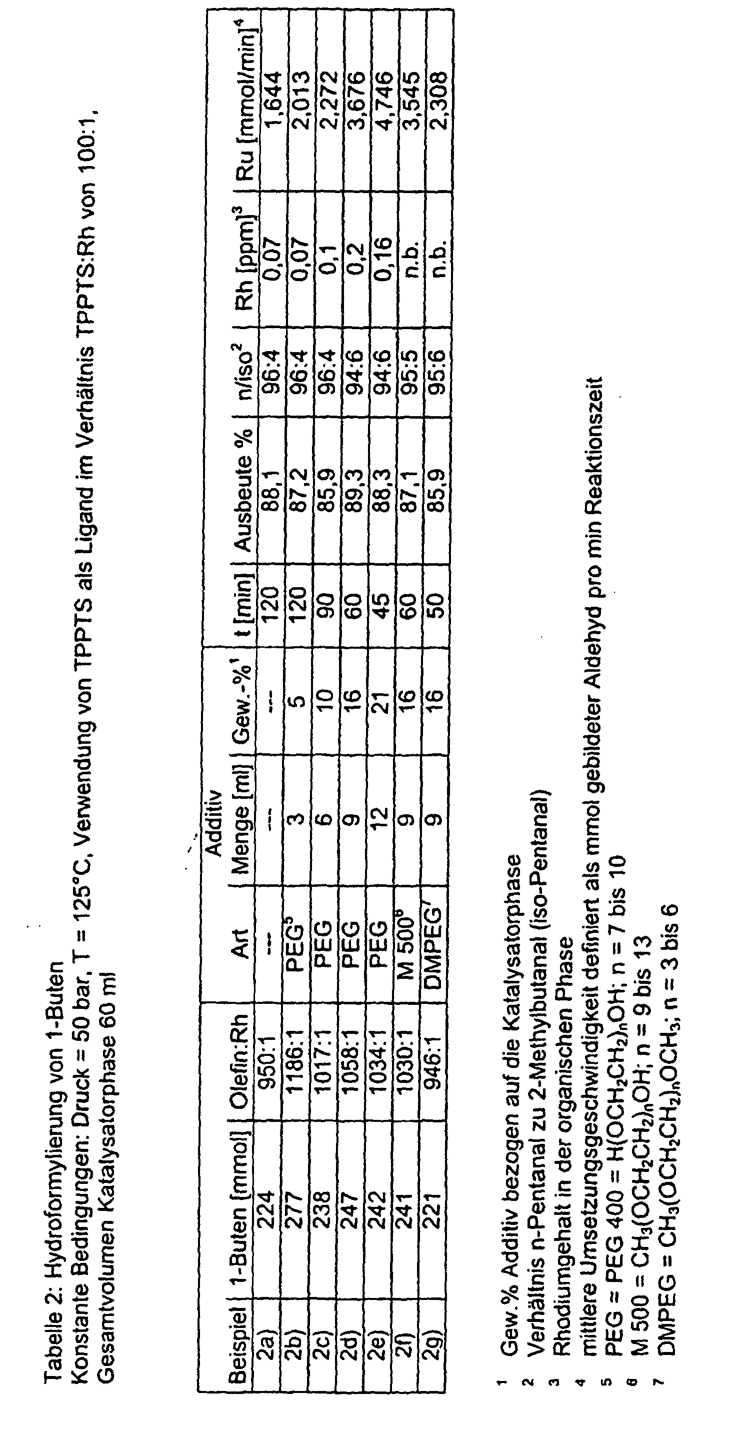

## Patentansprüche

1. Verfahren zur Herstellung von Aldehyden, **dadurch gekennzeichnet, daß** man Buten in Anwesenheit einer Rhodium und sulfonierte Triarylphosphine als Katalysator und von 8 bis 20 Gew.-% einer Verbindung der Formel (1) R(OCH₂CH₂)ₙOR¹ enthaltenden wäßrigen Phase, wobei die Triarylphosphine wenigstens drei -(SO₃)M-Reste enthalten, bei denen M gleich oder verschieden ist und M für H, ein Alkalimetallion, ein Ammoniumion, ein quaternäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

2. Verfahren zur Herstellung von Aldehyden, **dadurch gekennzeichnet, daß** man Penten in Anwesenheit einer Rhodium und sulfonierte Triarylphosphine als Katalysator und von 8 bis 30 Gew.-% einer Verbindung der Formel (1) R(OCH₂CH₂)ₙOR¹ enthaltenden wäßrigen Phase, wobei die Triarylphosphine wenigstens drei -(SO₃)M-Reste enthalten, bei denen M gleich oder verschieden ist und M für H, ein Alkalimetallion, ein Ammoniumion, ein quaternäres Ammoniumion, ein ½ Erdalkalimetallion oder ½ Zinkion steht, und wobei in der Formel (1) R für Wasserstoff, einen geradkettigen oder verzweigten Alkylrest mit 1 bis 4 Kohlenstoffatomen oder einen Hydroxyalkylrest mit 1 bis 4 Kohlenstoffatomen, R¹ für Wasserstoff oder einen Methylrest und n für eine ganze Zahl von 3 bis 50 steht, mit Wasserstoff und Kohlenmonoxid bei 20 bis 170°C und 1 bis 300 bar umsetzt.

3. Verfahren nach Anspruch 1 oder 2, **dadurch gekennzeichnet, daß** man als sulfonierte Triarylphosphine Verbindungen der Formel (2) 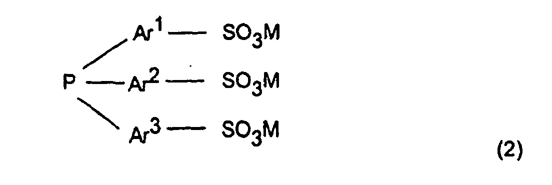 worin Ar¹, Ar² und Ar³ gleich oder verschieden sind und jeweils einen Phenyl - oder Naphthylrest bedeuten, und M gleich oder verschieden ist und für ein Alkalimetallion, ein Ammoniumion, ein quaternäres Ammoniumion oder ein ½ Erdalkalimetallion oder ½ Zinkion steht, einsetzt.

4. Verfahren nach einem oder mehreren der Ansprüche 1 bis 3, **dadurch gekennzeichnet, daß** man als sulfonierte Triarylphosphine ein trisulfoniertes Triphenylphosphin einsetzt.

5. Verfahren nach einem oder mehreren der Ansprüche 1 bis 3, **dadurch gekennzeichnet, daß** man als sulfoniertes Triarylphosphin Trinatrium-tri(m-sulfophenyl)phosphin einsetzt.

6. Verfahren nach einem oder mehreren der Ansprüche 1 bis 5, **dadurch gekennzeichnet, daß** man die wäßrige Phase entsprechend einer Menge von 2x10⁻⁶ bis 5x10⁻² Mol Rhodium pro Mol olefinische Verbindung einsetzt.

7. Verfahren nach einem oder mehreren der Ansprüche 1 bis 6, **dadurch gekennzeichnet, daß** man Rhodium und sulfonierte Triarylphosphine der Formel (2) im Molverhältnis 1 : 10 bis 1 : 1000 einsetzt.

8. Verfahren nach einem oder mehreren der Ansprüche 1 bis 7, **dadurch gekennzeichnet, daß** man Rhodium und sulfonierte Triarylphosphine der Formel (2) im Molverhältnis 1 : 50 bis 1 : 200 einsetzt.

9. Verfahren nach einem oder mehreren der Ansprüche 1 bis 8, **dadurch gekennzeichnet, daß** die wäßrige Phase bei Verwendung sulfonierter Triarylphosphine der Formel (2) 100 bis 1000 ppm Rhodium enthält.

10. Verfahren nach einem oder mehreren der Ansprüche 1 bis 9, **dadurch gekennzeichnet, daß** die wäßrige Phase bei Verwendung sulfonierter Triarylphosphine der Formel (2) 200 bis 500 ppm Rhodium enthält.

11. Verfahren nach einem oder mehreren der Ansprüche 1 bis 10, **dadurch gekennzeichnet, daß** die wäßrige Phase bei Verwendung sulfonierter Triarylphosphine der Formel (2) 300 bis 400 ppm Rhodium enthält.

12. Verfahren nach einem oder mehreren der Ansprüche 1 bis 11, **dadurch gekennzeichnet, daß** man als sulfonierte Triarylphosphine Verbindungen der Formel (3) 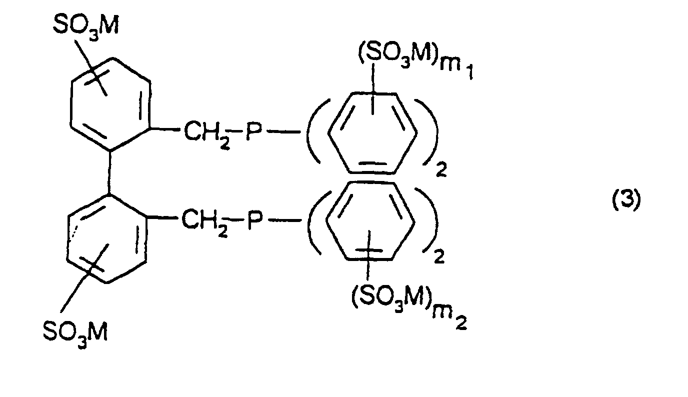 einsetzt, worin m₁ und m₂ unabhängig voneinander für 0 oder 1 steht und die Verbindungen der Formel (3) drei bis sechs -SO₃M-Gruppen, worin M die vorstehend genannte Bedeutung besitzt, enthalten.

13. Verfahren nach einem oder mehreren der Ansprüche 1 bis 12, **dadurch gekennzeichnet, daß** man als sulfonierte Triarylphosphine Verbindungen der Formel (4) 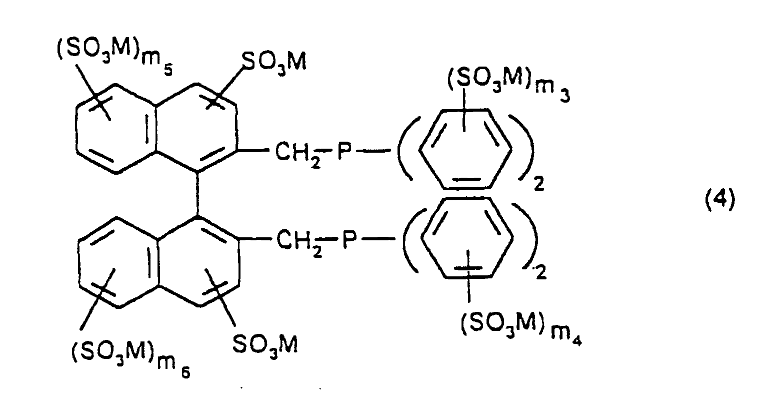 einsetzt, worin m₃, m₄, m₅ und m₆ unabhängig voneinander für 0 oder 1 steht und die Verbindungen der Formel (4) vier bis acht -SO₃M-Gruppen, worin M die vorstehend genannten Bedeutung besitzt, enthalten.

14. Verfahren nach einem oder mehreren der Ansprüche 1 bis 13, **dadurch gekennzeichnet, daß** man Rhodium und sulfonierte Triarylphosphine der Formel (3) oder (4) im Molverhältnis 1:5 bis 1:100 einsetzt.

15. Verfahren nach einem oder mehreren der Ansprüche 1 bis 14, **dadurch gekennzeichnet, daß** man Rhodium und sulfonierte Triarylphosphine der Formel (3) und (4) im Molverhältnis 1:5 bis 1:50 einsetzt.

16. Verfahren nach einem oder mehreren der Ansprüche 1 bis 15, **dadurch gekennzeichnet, daß** man Rhodium und sulfonierte Triarylphosphine der Formel (3) oder (4) im Molverhältnis 1:8 bis 1:15 einsetzt.

17. Verfahren nach einem oder mehreren der Ansprüche 1 bis 16, **dadurch gekennzeichnet, daß** die wäßrige Phase bei Verwendung sulfonierter Triarylphosphine der Formel (3) oder (4) 20 bis 500 ppm Rhodium enthält.

18. Verfahren nach einem oder mehreren der Ansprüche 1 bis 17, **dadurch gekennzeichnet, daß** die wäßrige Phase bei Verwendung sulfonierter Triarylphosphine der Formel (3) oder (4) 30 bis 150 ppm Rhodium enthält.

19. Verfahren nach einem oder mehreren der Ansprüche 1 bis 18, **dadurch gekennzeichnet, daß** die wäßrige Phase bei Verwendung sulfonierter Triarylphosphine der Formel (3) oder (4) 40 bis 100 ppm Rhodium enthält.

20. Verfahren nach einem oder mehreren der Ansprüche 1 bis 19, **dadurch gekennzeichnet, daß** man ein Polyethylenglykol der Formel H(OCH₂CH₂)ₙOH, worin n für eine ganze Zahl von 3 bis 50 steht, als Verbindung der Formel (1), einsetzt.

21. Verfahren nach einem oder mehreren der Ansprüche 1 bis 20, **dadurch gekennzeichnet, daß** man ein Polyethylenglykol der Formel H(OCH₂CH₂)ₙOH, worin n für eine ganze Zahl von 4 bis 30 steht, als Verbindung der Formel (1) einsetzt.

22. Verfahren nach einem oder mehreren der Ansprüche 1 bis 21, **dadurch gekennzeichnet, daß** man ein Polyethylenglykol der Formel H(OCH₂CH₂)ₙOH, worin n für eine ganze Zahl von 6 bis 12 steht, als Verbindung der Formel (1) einsetzt.

23. Verfahren nach einem oder mehreren der Ansprüche 1 bis 22, **dadurch gekennzeichnet, daß** man eine Verbindung der Formel R(OCH₂CH₂)ₙOH, worin R für einen Methylrest oder β-Hydroxypropylrest und n für eine ganze Zahl von 3 bis 50 steht, als Verbindungen der Formel (1) einsetzt.

24. Verfahren nach einem oder mehreren der Ansprüche 1 bis 23, **dadurch gekennzeichnet, daß** man eine Verbindung der Formel R(OCH₂CH₂)ₙOH, worin R für einen Methylrest oder β-Hydroxypropylrest und n für eine ganze Zahl von 4 bis 30 steht, als Verbindungen der Formel (1) einsetzt.

25. Verfahren nach einem oder mehreren der Ansprüche 1 bis 24, **dadurch gekennzeichnet, daß** man die Umsetzung bei 50 bis 150°C durchführt.

26. Verfahren nach einem oder mehreren der Ansprüche 1 bis 25, **dadurch gekennzeichnet, daß** man die Umsetzung bei 100 bis 140°C durchführt.

27. Verfahren nach einem oder mehreren der Ansprüche 1 bis 26, **dadurch gekennzeichnet, daß** man die Umsetzung bei 20 bis 150 bar durchführt.

28. Verfahren nach einem oder mehreren der Ansprüche 1 bis 27, **dadurch gekennzeichnet, daß** man die Umsetzung bei 30 bis 80 bar durchführt.

## Claims

1. A process for preparing aldehydes, which comprises reacting butene in the presence of an aqueous phase comprising rhodium and sulfonated triarylphosphines as catalyst and from 8 to 20% by weight of a compound of the formula (1) R(OCH₂CH₂)ₙOR¹, where the triarylphosphines contain at least three -(SO₃)M-groups, in which M are identical or different and are each hydrogen, an alkali metal ion, an ammonium ion, a quaternary ammonium ion, an ½ alkaline earth metal ion or ½ zinc ion, and where, in the formula (1), R is hydrogen, a straight-chain or branched alkyl radical having from 1 to 4 carbon atoms or a hydroxyalkyl radical having from 1 to 4 carbon atoms, R¹ is hydrogen or a methyl radical and n is an integer from 3 to 50, with hydrogen and carbon monoxide at from 20 to 170°C and from 1 to 300 bar.

2. A process for preparing aldehydes, which comprises reacting pentene in the presence of an aqueous phase comprising rhodium and sulfonated triarylphosphines as catalyst and from 8 to 30% by weight of a compound of the formula (1) R(OCH₂CH₂)ₙOR¹, where the triarylphosphines contain at least three -(SO₃)M-groups, in which M are identical or different and are each hydrogen, an alkali metal ion, an ammonium ion, a quaternary ammonium ion, an ½ alkaline earth metal ion or ½ zinc ion, and where, in the formula (1), R is hydrogen, a straight-chain or branched alkyl radical having from 1 to 4 carbon atoms or a hydroxyalkyl radical having from 1 to 4 carbon atoms, R¹ is hydrogen or a methyl radical and n is an integer from 3 to 50, with hydrogen and carbon monoxide at from 20 to 170°C and from 1 to 300 bar.

3. The process as claimed in claim 1 or 2, wherein the sulfonated triarylphosphines used are compounds of the formula (2) 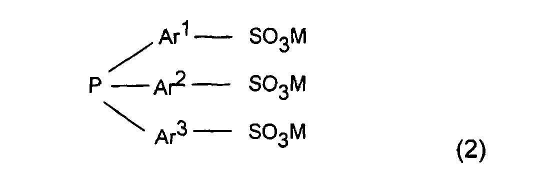 where Ar¹, Ar² and Ar³ are identical or different and are each a phenyl or naphthyl radical and M are identical or different and are each an alkali metal ion, an ammonium ion, a quaternary ammonium ion or a ½ alkaline earth metal ion or ½ zinc ion.

4. The process as claimed in one or more of claims 1 to 3, wherein the sulfonated triarylphosphine used is a trisulfonated triphenylphosphine.

5. The process as claimed in one or more of claims 1 or 3, wherein the sulfonated triarylphosphine used is trisodium tri(m-sulfophenyl)phosphine.

6. The process as claimed in one or more of claims 1 to 5, wherein the aqueous phase is used in an amount corresponding to from 2 x 10⁻⁶ to 5 x 10⁻² mol of rhodium per mol of olefinic compound.

7. The process as claimed in one or more of claims 1 to 6, wherein rhodium and sulfonated triarylphosphines of the formula (2) are used in a molar ratio of from 1:10 to 1:1000.

8. The process as claimed in one or more of claims 1 to 7, wherein rhodium and sulfonated triarylphosphines of the formula (2) are used in a molar ratio of from 1:50 to 1:200.

9. The process as claimed in one or more of claims 1 to 8, wherein the aqueous phase contains from 100 to 1000 ppm of rhodium when using sulfonated triarylphosphines of the formula (2).

10. The process as claimed in one or more of claims 1 to 9, wherein the aqueous phase contains from 200 to 500 ppm of rhodium when using sulfonated triarylphosphines of the formula (2).

11. The process as claimed in one or more of claims 1 to 10, wherein the aqueous phase contains from 300 to 400 ppm of rhodium when using sulfonated triarylphosphines of the formula (2).

12. The process as claimed in one or more of claims 1 to 11, wherein the sulfonated triarylphosphines used are compounds of the formula (3) 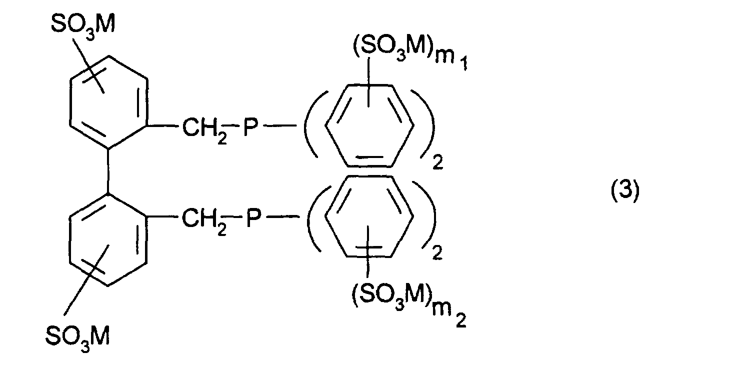 where m₁ and m₂ are, independently of one another, 0 or 1 and the compounds of the formula (3) contain from three to six -SO₃M groups, where M is as defined above.

13. The process as claimed in one or more of claims 1 to 12, wherein the sulfonated triarylphosphines used are compounds of the formula (4) 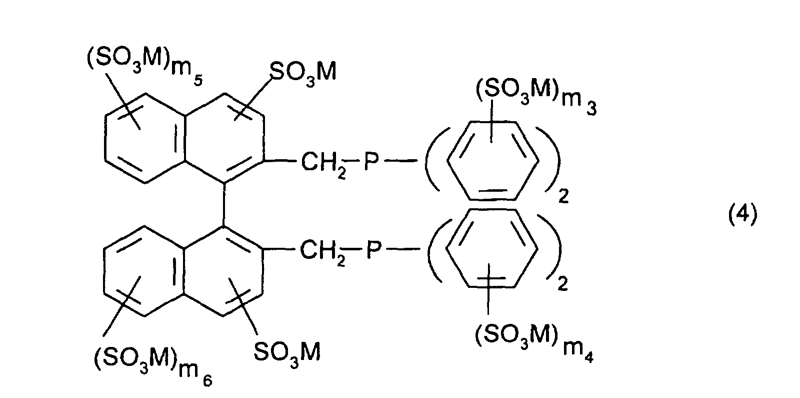 where m₃, m₄, m₅ and m₆ are, independently of one another, 0 or 1 and the compounds of the formula (4) have from four to eight -SO₃M groups, where M is as defined above.

14. The process as claimed in one or more of claims 1 to 13, wherein rhodium and sulfonated triarylphosphines of the formula (3) or (4) are used in a molar ratio of 1:5 to 1:100.

15. The process as claimed in one or more of claims 1 to 14, wherein rhodium and sulfonated triarylphosphines of the formula (3) or (4) are used in a molar ratio of 1:5 to 1:50.

16. The process as claimed in one or more of claims 1 to 15, wherein rhodium and sulfonated triarylphosphines of the formula (3) or (4) are used in a molar ratio of 1:8 to 1:15.

17. The process as claimed in one or more of claims 1 to 16, wherein the aqueous phase contains from 20 to 500 ppm of rhodium when using sulfonated triarylphosphines of the formula (3) or (4).

18. The process as claimed in one or more of claims 1 to 17, wherein the aqueous phase contains from 30 to 150 ppm of rhodium when using sulfonated triarylphosphines of the formula (3) or (4).

19. The process as claimed in one or more of claims 1 to 18, wherein the aqueous phase contains from 40 to 100 ppm of rhodium when using sulfonated triarylphosphines of the formula (3) or (4).

20. The process as claimed in one or more of claims 1 to 19, wherein the compound of the formula (1) used is a polyethylene glycol of the formula H(OCH₂CH₂)ₙOH, where n is an integer from 3 to 50.

21. The process as claimed in one or more of claims 1 to 20, wherein the compound of the formula (1) used is a polyethylene glycol of the formula H(OCH₂CH₂)ₙOH, where n is an integer from 4 to 30.

22. The process as claimed in one or more of claims 1 to 21, wherein the compound of the formula (1) used is a polyethylene glycol of the formula H(OCH₂CH₂)ₙOH, where n is an integer from 6 to 12.

23. The process as claimed in one or more of claims 1 to 22, wherein the compound of the formula (1) used is a compound of the formula R(OCH₂CH₂)ₙOH, where R is a methyl radical or β-hydroxypropyl radical and n is an integer from 3 to 50.

24. The process as claimed in one or more of claims 1 to 23, wherein the compound of the formula (1) used is a compound of the formula R(OCH₂CH₂)ₙOH, where R is a methyl radical or β-hydroxypropyl radical and n is an integer from 4 to 30.

25. The process as claimed in one or more of claims 1 to 24, wherein the reaction is carried out at from 50 to 150°C.

26. The process as claimed in one or more of claims 1 to 25, wherein the reaction is carried out at from 100 to 140°C.

27. The process as claimed in one or more of claims 1 to 26, wherein the reaction is carried out at from 20 to 150 bar.

28. The process as claimed in one or more of claims 1 to 27, wherein the reaction is carried out at from 30 to 80 bar.

## Revendications

1. Procédé pour la préparation d'aldéhydes, **caractérisé en ce qu'**on transforme du butène en présence d'une phase aqueuse contenant du rhodium et des triarylphoshines sulfonées comme catalyseur et 8 à 20% en poids d'un composé de formule (1) R(OCH₂CH₂)ₙOR¹, les triarylphosphines contenant au moins trois radicaux -(SO₃)M, dans lesquels M est indentique ou différent et M représente H, un ion de métal alcalin, un ion d'ammonium, un ion d'ammonium quaternaire, un ½ ion de métal alcalino-terreux ou ½ ion de zinc, et, dans la formule (1), R représentant un hydrogène, un radical alkyle linéaire ou ramifié comprenant 1 à 4 atomes de carbone ou un radical hydroxyalkyle comprenant 1 à 4 atomes de carbone, R¹ représentant un hydrogène ou un radical méthyle et n représentant un nombre entier de 3 à 50, avec de l'hydrogène et du monoxyde de carbone à 20 jusqu'à 170°C et 1 à 300 bars.

2. Procédé pour la préparation d'aldéhydes, **caractérisé en ce qu'**on transforme du pentène en présence d'une phase aqueuse contenant du rhodium et des triarylphoshines sulfonées comme catalyseur et 8 à 30% en poids d'un composé de formule (1) R(OCH₂CH₂)ₙOR¹, les triarylphosphines contenant au moins trois radicaux -(SO₃)M, dans lesquels M est indentique ou différent et M représente H, un ion de métal alcalin, un ion d'ammonium, un ion d'ammonium quaternaire, un ½ ion de métal alcalino-terreux ou ½ ion de zinc, et, dans la formule (1), R représentant un hydrogène, un radical alkyle linéaire ou ramifié comprenant 1 à 4 atomes de carbone ou un radical hydroxyalkyle comprenant 1 à 4 atomes de carbone, R¹ représentant un hydrogène ou un radical méthyle et n représentant un nombre entier de 3 à 50, avec de l'hydrogène et du monoxyde de carbone à 20 jusqu'à 170°C et 1 à 300 bars.

3. Procédé selon la revendication 1 ou 2, **caractérisé en ce qu'**on utilise comme triarylphosphines sulfonées des composés de formule (2) 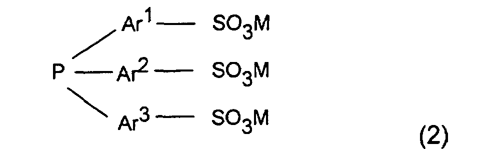 dans laquelle Ar¹, Ar² et Ar³ sont identiques ou différents et signifient à chaque fois un radical phényle ou naphthyle, et M est identique ou différent et représente un ion de métal alcalin, un ion d'ammonium, un ion d'ammonium qutemaire ou un ½ ion de métal alcalino-terreux ou un ½ ion de zinc.

4. Procédé selon l'une quelconque ou plusieurs des revendications 1 à 3, **caractérisé en ce qu'**on utilise une triphénylphoshine trisulfonée comme triarylphosphines sulfonées.

5. Procédé selon l'une quelconque ou plusieurs des revendications 1 à 3, **caractérisé en ce qu'**on utilise de la tri(m-sulfophényl)phosphine trisodique comme triarylphosphine sulfonée.

6. Procédé selon l'une quelconque ou plusieurs des revendications 1 à 5, **caractérisé en ce qu'**on utilise la phase aqueuse correspondant à une quantité de 2x10⁻⁶ à 5x10⁻² mole de rhodium par mole de composé oléfinique.

7. Procédé selon l'une quelconque ou plusieurs des revendications 1 à 6, **caractérisé en ce qu'**on utilise le rhodium et les triarylphosphines sulfonées de fomule (2) dans un rapport molaire de 1 :10 à 1 :1000.

8. Procédé selon l'une quelconque ou plusieurs des revendications 1 à 7, **caractérisé en ce qu'**on utilise le rhodium et les triarylphosphines sulfonées de fomule (2) dans un rapport molaire de 1:50 à 1:200.

9. Procédé selon l'une quelconque ou plusieurs des revendications 1 à 8, **caractérisé en ce que** la phase aqueuse contient 100 à 1000 ppm de rhodium dans le cas de l'utilisation de triarylphosphines sulfonées de formule (2).

10. Procédé selon l'une quelconque ou plusieurs des revendications 1 à 9, **caractérisé en ce que** la phase aqueuse contient 200 à 500 ppm de rhodium dans le cas de l'utilisation de triarylphosphines sulfonées de formule (2).

11. Procédé selon l'une quelconque ou plusieurs des revendications 1 à 10, **caractérisé en ce que** la phase aqueuse contient 300 à 400 ppm de rhodium dans le cas de l'utilisation de triarylphosphines sulfonées de formule (2).

12. Procédé selon l'une quelconque ou plusieurs des revendications 1 à 11, **caractérisé en ce qu'**on utilise comme triarylphosphines sulfonées des composés de fomule (3) 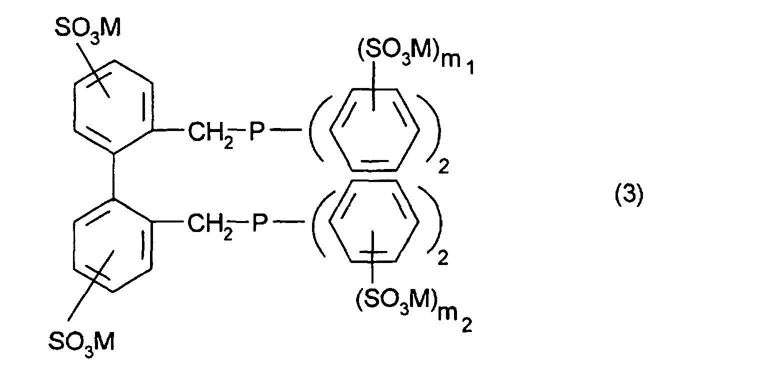 dans laquelle m₁ et m₂ représentent, indépendamment l'un de l'autre, 0 ou 1 et les composés de formule (3) présentent trois à six groupements -SO₃M, M présentant la signification susmentionnée.

13. Procédé selon l'une quelconque ou plusieurs des revendications 1 à 12, **caractérisé en ce qu'**on utilise comme triarylphosphines sulfonées des composés de formule (4) 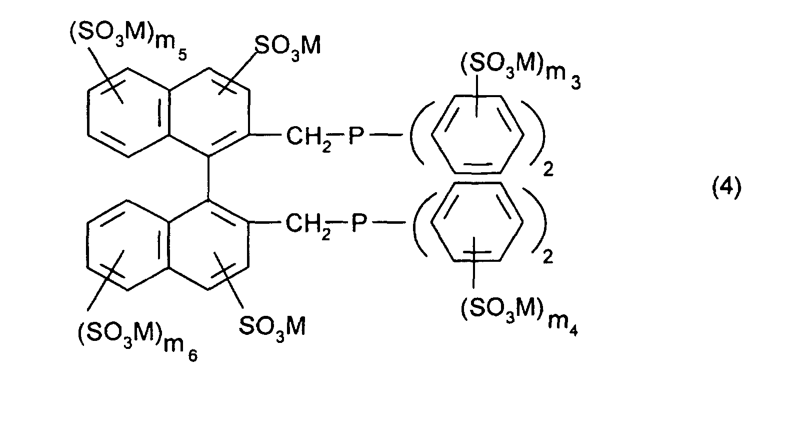 dans laquelle m₃, m₄, m₅ et m₆ représentent, indépendamment l'un de l'autre, 0 ou 1 et les composés de formule (4) présentent quatre à huit groupements -SO₃M, M présentant la signification susmentionnée.

14. Procédé selon l'une quelconque ou plusieurs des reventications 1 à 13, **caractérisé en ce qu'**on utilise le rhodium et les triarylphosphines sulfonées de formule (3) ou (4) dans un rapport molaire de 1:5 à 1:100.

15. Procédé selon l'une quelconque ou plusieurs des reventications 1 à 14, **caractérisé en ce qu'**on utilise le rhodium et les triarylphosphines sulfonées de formule (3) ou (4) dans un rapport molaire de 1:5 à 1:50.

16. Procédé selon l'une quelconque ou plusieurs des reventications 1 à 15, **caractérisé en ce qu'**on utilise le rhodium et les triarylphosphines sulfonées de formule (3) ou (4) dans un rapport molaire de 1:8 à 1:15.

17. Procédé selon l'une quelconque ou plusieurs des revendications 1 à 16, **caractérisé en ce que** la phase aqueuse contient 20 à 500 ppm de rhodium dans le cas de l'utilisation de triarylphosphines sulfonées de formule (3) ou (4).

18. Procédé selon l'une quelconque ou plusieurs des revendications 1 à 17, **caractérisé en ce que** la phase aqueuse contient 30 à 150 ppm de rhodium dans le cas de l'utilisation de triarylphosphines sulfonées de formule (3) ou (4).

19. Procédé selon l'une quelconque ou plusieurs des revendications 1 à 18, **caractérisé en ce que** la phase aqueuse contient 40 à 100 ppm de rhodium dans le cas de l'utilisation de triarylphosphines sulfonées de formule (3) ou (4).

20. Procédé selon l'une quelconque ou plusieurs des revendications 1 à 19, **caractérisé en ce qu'**on utilise un polyéthylèneglycol de formule H(OCH₂CH₂)ₙOH dans laquelle n représente un nombre entier de 3 à 50 comme composé de formule (1).

21. Procédé selon l'une quelconque ou plusieurs des revendications 1 à 20, **caractérisé en ce qu'**on utilise un polyéthylèneglycol de formule H(OCH₂CH₂)ₙOH dans laquelle n représente un nombre entier de 4 à 30 comme composé de formule (1).

22. Procédé selon l'une quelconque ou plusieurs des revendications 1 à 21, **caractérisé en ce qu'**on utilise un polyéthylèneglycol de formule H(OCH₂CH₂)ₙOH dans laquelle n représente un nombre entier de 6 à 12 comme composé de formule (1).

23. Procédé selon l'une quelconque ou plusieurs des revendications 1 à 22, **caractérisé en ce qu'**on utilise un composé de formule R(OCH₂CH₂)ₙOH dans laquelle R représente un radical méthyle ou un radical β-hydroxypropyle et n représente un nombre entier de 3 à 50 comme composés de formule (1).

24. Procédé selon l'une quelconque ou plusieurs des revendications 1 à 23, **caractérisé en ce qu'**on utilise un composé de formule R(OCH₂CH₂)ₙOH dans laquelle R représente un radical méthyle ou un radical β-hydroxypropyle et n représente un nombre entier de 4 à 30 comme composés de formule (1).

25. Procédé selon l'une quelconque ou plusieurs des revendications 1 à 24, **caractérisé en ce qu'**on effectue la transformation à 50 jusqu'à 150°C.

26. Procédé selon l'une quelconque ou plusieurs des revendications 1 à 25, **caractérisé en ce qu'**on effectue la transformation à 100 jusqu'à 140°C.

27. Procédé selon l'une quelconque ou plusieurs des revendications 1 à 26, **caractérisé en ce qu'**on effectue la transformation à 20 jusqu'à 150 bars.

28. Procédé selon l'une quelconque ou plusieurs des revendications 1 à 27, **caractérisé en ce qu'**on effectue la transformation à 30 jusqu'à 80 bars.
